# FASCICULE DE BREVET EUROPEEN

(11) **EP 1 165 481 B1**
(45) Date de publication et mention de la délivrance du brevet: **17.09.2003**
(21) Numéro de dépôt: 00915243.0
(22) Date de dépôt: 29.03.2000
(51) Int. Cl.: C07C 51/21, C07C 51/31

(54) **PROCEDE D'OXYDATION DE CYCLOALCANES, DE CYCLOALCANOLS ET/OU DE CYCLOALCANONES**
VERFAHREN ZUR OXIDATION VON CYCLOALKANEN, CYCLOALKOHOLEN UND/ODER CYCLOKETONEN
PROCESS FOR THE OXIDATION OF CYCLOALKANES, CYCLOALCOHOLS AND/OR CYCLOKETONES

(30) Priorité: 30.03.1999 FR 9904203
(43) Date de publication de la demande: 02.01.2002
(73) Titulaire: Rhodia Polyamide Intermediates, 69192 Saint-Fons (FR)
(72) Inventeur: FACHE, Eric, F-69300 Caluire et Cuire (FR); COSTANTINI, Michel, F-69003 Lyon (FR)
(74) Mandataire: Esson, Jean-Pierre
(86) Numéro de dépôt international: FR0000786
(87) Numéro de publication internationale: WO00059858

(56) Documents cités:
- EP-A- 0 870 751
- GB-A- 415 172
- GB-A- 942 415
- GB-A- 1 086 951
- DATABASE WPI Section Ch, Week 199727 Derwent Publications Ltd., London, GB; Class A41, AN 1997-296571 XP002126081 & SU 982 319 A (MONOMERS RES DES INST), 10 novembre 1996 (1996-11-10)

## Description

La présente invention concerne l'oxydation par l'oxygène ou un gaz en contenant, de cycloalcanes en acides carboxyliques, alcools et/ou cétones correspondants ou de cycloalcanols et/ou de cycloalcanones en acides carboxyliques correspondants.

L'oxydation directe par l'oxygène des hydrocarbures, plus particulièrement des cycloalcanes, en présence d'un catalyseur, est un procédé qui a été étudié depuis longtemps. En effet, il y aurait des avantages évidents à éviter l'emploi d'un oxydant comme l'acide nitrique, utilisé dans une des étapes des procédés industriels actuels, ce qui épargnerait le traitement des oxydes d'azote générés.

Dans les nombreuses variantes d'un tel procédé d'oxydation catalytique par l'oxygène, le cobalt est le catalyseur le plus fréquemment préconisé.

Ainsi le brevet américain US-A-2 223 493, publié en décembre 1940, décrit l'oxydation d'hydrocarbures cycliques en diacides correspondants, en phase liquide comportant généralement de l'acide acétique, à une température d'au moins 60°C, à l'aide d'un gaz contenant de l'oxygène et en présence d'un catalyseur d'oxydation tel qu'un composé du cobalt.

Le brevet américain US-A-4 902 827, publié en février 1990, décrit un perfectionnement à l'oxydation par l'air du cyclohexane en acide adipique, en phase liquide comportant l'acide acétique, à une température de 80°C à 160°C et en présence d'un catalyseur d'oxydation comprenant un composé soluble du cobalt et un composé soluble du zirconium et/ou de l'hafnium.

Plus récemment, il a été préconisé dans le brevet EP-A-0 694 333 de mettre en oeuvre dans le cadre de l'oxydation des hydrocarbures par l'oxygène, un catalyseur comprenant un sel cobaltique et un sel ferrique.

Il a également été proposé par le brevet russe SU19792722487 un procédé d'oxydation de benzène ou d'alkylbenzènes en di ou tri-acides carboxyliques en présence d'un catalyseur métallique à base de chrome, de cobalt, de manganèse et de nickel.
En outre, le brevet EP870751 décrit l'oxydation de cycloalcanes en présence d'un catlyseur à base de chrome et de cobalt.

Comme autre catalyseur usuel de cette réaction d'oxydation, on peut citer le manganèse.

Pour des raisons économiques et également pour faciliter la purification des produits obtenus, il est préférable de travailler avec la concentration en catalyseur la plus faible possible. Ainsi, le manganèse est un catalyseur interessant dans les procédés d'oxydation du cyclohexane.

Il s'avère néanmoins que les sélectivités obtenues avec les systèmes catalytiques utilisés dans les procédés antérieurs décrits précédemment doivent encore être améliorées.

C'est ce que se propose de réaliser la présente invention. Elle consiste plus précisément en un procédé d'oxydation de cycloalcanes, de cycloalcanols et/ou de cycloalcanones à l'aide d'oxygène ou d'un gaz en contenant, en phase liquide et en présence d'un catalyseur dissous dans le milieu réactionnel, caractérisé en ce que le catalyseur comprend au moins un composé soluble du manganèse et au moins un composé soluble du chrome.

Les cycloalcanes, notamment ceux qui ont un cycle ayant de 5 à 12 atomes de carbone, sont certainement les plus importants, car leur oxydation conduit aux diacides carboxyliques ou aux cycloalcanols et cycloalcanones intermédiaires.

Le cycloalcane le plus intéressant est le cyclohexane dont l'oxydation conduit à l'acide adipique, l'un des composés de base du polyamide 6-6, mais peut aussi fournir la cyclohexanone conduisant au caprolactame et donc au polyamide 6.

Le présent procédé peut également être utilisé pour l'oxydation des alcools ou cétones intermédiaires, notamment les cycloalcanols et cycloalcanones ayant de 5 à 12 atomes de carbone, pour préparer les diacides carboxyliques correspondants.
Dans ce qui suit, le procédé sera plus particulièrement décrit pour l'oxydation des cycloalcanes, et de manière privilégiée pour l'oxydation du cyclohexane.

Le système catalytique comprenant des composés du manganèse, du chrome permet de préparer directement l'acide adipique avec une bonne sélectivité, lorsque l'on réalise l'oxydation du cyclohexane ; cette capacité est évidemment très avantageuse.

Le système catalytique comprend au moins un composé du manganèse soluble dans le milieu réactionnel, choisi par exemple de manière non limitative parmi le chlorure de manganèse, le bromure de manganèse, le nitrate de manganèse et les carboxylates de manganèse comme l'acétate de manganèse tétrahydraté, le propionate de manganèse, l'adipate de manganèse, le glutarate de manganèse, le succinate de manganèse.

Le catalyseur comprend également au moins un composé du chrome soluble dans le milieu réactionnel, choisi par exemple de manière non limitative parmi le chlorure de chrome, le bromure de chrome, le nitrate de chrome et les carboxylates de chrome comme l'acétate de chrome, le propionate de chrome, l'adipate de chrome, le glutarate de chrome, le succinate de chrome.

Enfin, Le catalyseur peut comprendre également au moins un composé du zirconium et/ou de l'hafnium soluble dans le milieu réactionnel, choisi par exemple de manière non limitative parmi le chlorure de zirconium, le bromure de zirconium, le nitrate de zirconium et les carboxylates de zirconium comme l'acétate de zirconium, le propionate de zirconium, l'adipate de zirconium, le glutarate de zirconium, le succinate de zirconium et le chlorure d'hafnium, le bromure d'hafnium, le nitrate d'hafnium et les carboxylates d'hafnium comme l'acétate d'hafnium, le propionate d'hafnium, l'adipate d'hafnium, le glutarate d'hafnium, le succinate d'hafnium.

Les rapports molaires entre le chrome et le manganèse dans le système catalytique peuvent varier dans de larges limites. On peut ainsi mettre en oeuvre des rapports molaires Cr/Mn compris avantageusement entre 0,00001 et 100, préférentiellement entre 0,001 et 10.

La quantité de zirconium quand il est présent peut varier dans des rapports molaires par rapport au manganèse, semblables à ceux indiqués ci-dessus pour le chrome.

Le catalyseur peut être obtenu in situ en chargeant les composés du manganèse, du chrome et éventuellement, du zirconium dans le milieu réactionnel. Il peut également être préparé extemporanément par mélange desdits composés dans les proportions nécessaires pour obtenir les rapports molaires Cr/Mn et éventuellement Zr/Mn souhaités. De préférence ce mélange est réalisé en utilisant un solvant, avantageusement un solvant de même nature qe celui utilisé pour la réaction d'oxydation, ou directement dans ce solvant.

La quantité de catalyseur, exprimée en pourcentage pondéral d'éléments manganèse, chrome et éventuellement, zirconium par rapport au mélange réactionnel, se situe généralement entre 0,0001 et 5 %, avatageusement entre 0,001 et 1 %, sans que ces valeurs soient critiques. Il s'agit cependant d'avoir une activité suffisante sans toutefois utiliser des quantités trop importantes. En effet, le catalyseur devra être séparé du milieu réactionnel final et recyclé.

Il est avantageux de mettre en oeuvre également un composé initiateur de la réaction d'oxydation. Les initiateurs sont souvent des hydroperoxydes, comme par exemple l'hydroperoxyde de cyclohexyle ou l'hydroperoxyde de tertiobutyle. Ce sont également des cétones ou des aldéhydes, comme par exemple la cyclohexanone qui est l'un des composés formés lors de l'oxydation du cyclohexane ou l'acétaldéhyde. Généralement l'initiateur représente de 0,01 % à 20 % en poids du poids du mélange réactionnel mis en oeuvre, sans que ces proportions aient une valeur critique. L'initiateur est surtout utile lors du démarrage de l'oxydation et lorsque l'on réalise l'oxydation du cyclohexane à une température inférieure à 120°C. Il peut être introduit dès le début de la réaction.

Le milieu réactionnel liquide contient préférentiellement un solvant au moins partiel de l'acide carboxylique et/ou de l'alcool et/ou de la cétone dont la préparation est visée par la mise en oeuvre du procédé de l'invention. Ce solvant peut être de nature très variée dans la mesure où il n'est pas sensiblement oxydable dans les conditions réactionnelles. Il peut être notamment choisi parmi les solvants protiques polaires et les solvants aprotiques polaires. Comme solvants protiques polaires, on peut citer par exemple les acides carboxyliques ne possédant que des atomes d'hydrogène primaires ou secondaires, en particulier les acides aliphatiques ayant de 2 à 9 atomes de carbone, les acides perfluoroalkylcarboxyliques tel que l'acide trifluoroacétique, les alcools tels que le tertiobutanol. Comme solvants aprotiques polaires, on peut citer par exemple les esters d'alkyle inférieur (= radical alkyle ayant de 1 à 4 atomes de carbone) d'acides carboxyliques, en particulier des acides carboxyliques aliphatiques ayant de 2 à 9 atomes de carbone ou des acides perfluoroalkylcarboxyliques, la tétraméthylène sulfone (ou sulfolane), l'acétonitrile, les hydrocarbures halogénés tel que le dichlorométhane, les cétones telles que l'acétone.

L'acide acétique est utilisé de préférence comme solvant de la réaction d'oxydation du cyclohexane. Il est commode de mettre en oeuvre un catalyseur dont les constituants manganèse et chrome soient sous la forme de composés dérivant de l'acide carboxylique utilisé comme solvant, dans la mesure où lesdits composés sont solubles dans le milieu réactionnel. Les acétates de manganèse et de chrome sont donc utilisés de préférence, notamment pour cette raison.

Le solvant, tel que défini précédemment, représente généralement de 1 % à 99 % en poids du milieu réactionnel, de préférence de 10 % à 90 % et encore plus préférentiellement de 20 % à 80 %.

L'oxydation peut également être mise en oeuvre en présence d'eau introduite dès le stade initial du procédé.

La température à laquelle est réalisée la réaction d'oxydation est variable, notamment selon le substrat mis en oeuvre. Elle est généralement comprise entre 50°C et 200°C et de préférence entre 80 °C et 140°C.

La pression n'est pas un paramètre critique du procédé. Elle peut être inférieure, égale ou supérieure à la pression atmosphérique. Généralement elle se situera entre 0,1 MPa (1 bar) et 20 MPa (200 bar), sans que ces valeurs soient impératives.

On peut utiliser de l'oxygène pur, de l'air, de l'air enrichi ou appauvri en oxygène ou encore de l'oxygène dilué par un gaz inerte.

Les exemples qui suivent illustrent l'invention.

### EXEMPLE 1 COMPARATIF

Dans un autoclave de 125 ml en titane, muni de moyens de chauffage par collier chauffant, d'une turbine et de moyens d'introduction de gaz et de régulation de pression, on charge :
- 21,25 g (253 mmol) de cyclohexane
- 27,35 g d'acide acétique
- 0,26 g (2,65 mmol) de cyclohexanone
- 0,0143 g (0,057 mmol de Co) d'acétate de Cobalt tétrahydraté

Après fermeture du réacteur, on agite à 1000 tours par minute, on crée une pression d'air (100 bar à 20°C) et on chauffe. La température atteint 105°C dans la masse en 10 min et on maintient cette température pendant encore 170 min.

Après refroidissement et dépressurisation, le mélange réactionnel est constitué de deux phases liquides que l'on homogénéise par addition d'acide acétique.

Le mélange homogène ainsi obtenu est dosé par chromatographie en phase gazeuse.

On obtient les résultats suivants :
- taux de transformation (TT) du cyclohexane : < 1 %
Ce résultat démontre que le cobalt n'est pas un bon catalyseur à la concentration testée

### EXEMPLE 2 COMPARATIF

On répète l'exemple 1 dans le même appareillage et dans les mêmes conditions opératoires, mais en remplaçant l'acétate de cobalt hydraté par 0,061 mmol de Mn sous forme d'acétate de manganèse tétrahydraté (0,015g). Le temps de réacton est de 170 min.

On obtient les résultats suivants :
- taux de transformation (TT) du cyclohexane : 15,3 %
- ST en cyclohexanol par rapport au cyclohexane transformé : 24,5 %
- ST en cyclohexanone par rapport au cyclohexane transformé : 0,0 %
- ST en acide adipique par rapport au cyclohexane transformé : 48,4 %
- ST en acide adipique + cyclohexanone + cyclohexanol par rapport au cyclohexane transformé : 72,9 %
- rapport molaire acide adipiqueltotal des diacides formés : 77,6 %
- ST en autres composés (butyrolactone, valérolactone, acide hydroxyadipique, acide hydroxycaproïque): 13,2 %

### EXEMPLE 3

On répète l'exemple 2 dans le même appareillage et dans les mêmes conditions opératoires, mais en ajoutant au catalyseur 0,011 g d'acétate de chrome (0,04 mmol de Cr).

On obtient les résultats suivants :
- taux de transformation (TT) du cyclohexane : 11,3 %
- ST en cyclohexanol par rapport au cyclohexane transformé : 10,2 %
- ST en cyclohexanone par rapport au cyclohexane transformé : 0,0 %
- ST en acide adipique par rapport au cyclohexane transformé : 65,5 %
- ST en acide adipique + cyclohexanone + cyclohexanol par rapport au cyclohexane transformé : 75,7 %
- rapport molaire acide adipique/total des diacides formés : 78,9 %
- ST en autres composés : 6,7 %

### EXEMPLE 4

On répète l'exemple 2 dans le même appareillage et dans les mêmes conditions opératoires, mais en ajoutant 0,0031 g d'acétate de Cr au lieu de 0,011g.

On obtient les résultats suivants :
- taux de transformation (TT) du cydohexane : 13,4 %
- ST en cyclohexanol par rapport au cyclohexane transformé : 16,2 %
- ST en cyclohexanone par rapport au cyclohexane transformé : 0 %
- ST en acide adipique par rapport au cyclohexane transformé : 58,5 %
- ST en acide adipique + cyclohexanone + cyclohexanol par rapport au cyclohexane transformé : 74,7 %
- rapport molaire acide adipique/total des diacides formés : 79,0 %
- ST en autres composés : 9,8 %

### EXEMPLE 5

On répète l'exemple 3 dans le même appareillage et dans les mêmes conditions opératoires, mais en ajoutant 15 ppm de Zr sous forme d'acétate de zirconium.Le temps de réaction est de 60 min.

On obtient les résultats suivants :
- taux de transformation (TT) du cyclohexane : 14,0 %
- ST en cyclohexanol par rapport au cyclohexane transformé : 9,8 %
- ST en cyclohexanone par rapport au cyclohexane transformé : 2,5 %
- ST en acide adipique par rapport au cyclohexane.transformé : 64,5 %
- ST en acide adipique + cyclohexanone + cyclohexanol par rapport au cyclohexane transformé : 76,8 %
- rapport molaire acide adipique/total des diacides formés : 78,7 %
- ST en autres composés : 5,7 %

## Revendications

1. Procédé d'oxydation de cycloalcanes de cycloalcanols et/ou de cycloalcanones en acide carboxylique, à l'aide d'oxygène ou d'un gaz en contenant, en phase liquide dans un solvant choisi parmi les solvants protiques polaires et les solvants aprotiques polaires et en présence d'un catalyseur dissous dans le milieu réactionnel, **caractérisé en ce que** le catalyseur comprend un composé soluble du manganèse, et un composé soluble du chrome.

2. Procédé selon la revendication 1, **caractérisé en ce que** le cycloalcane utilisé comme substrat de départ est choisi parmi les cycloalcanes qui ont un cycle ayant de 5 à 12 atomes de carbone, et est de préférence le cyclohexane.

3. Procédé selon la revendication 1, **caractérisé en ce que** les cycloalcanols et cycloalcanones utilisés comme substrat de départ sont choisis parmi ceux qui ont un cycle ayant de 5 à 12 atomes de carbone, et sont de préférence le cyclohexanol et/ou la cyclohexanone.

4. Procédé selon l'une des revendications 1 à 3, **caractérisé en ce que** le catalyseur comprend au moins un composé du manganèse soluble dans le milieu réactionnel, choisi parmi le chlorure de manganèse, le bromure de manganèse, le nitrate de manganèse et les carboxylates de manganèse comme l'acétate de manganèse tétrahydraté, le propionate de manganèse, l'adipate de manganèse, le glutarate de manganèse, le succinate de manganèse.

5. Procédé selon l'une des revendications 1 à 4, **caractérisé en ce que** le catalyseur comprend au moins un composé du chrome soluble dans le milieu réactionnel, choisi parmi le chlorure de chrome, le bromure de chrome, le nitrate de chrome et les carboxylates de chrome comme l'acétate de chrome, le propionate de chrome, l'adipate de chrome, le glutarate de chrome, le succinate de chrome.

6. Procédé selon l'une des revendications précédentes, **caractérisé en ce que** le catalyseur comprend un composé soluble du zirconium ou d'hafnium

7. Procédé selon la revendication 6, **caractérisé en ce que** le composé soluble du zirconium dans le milieu réactionnel est choisi parmi le chlorure de zirconium, le bromure de zirconium, le nitrate de zirconium et les carboxylates de zirconium comme l'acétate de zirconium, le propionate de zirconium, l'adipate de zirconium, le glutarate de zirconium, le succinate de zirconium.

8. Procédé selon la revendication 6, **caractérisé en ce que** le composé soluble du hafnium dans le milieu réactionnel est choisi parmi le chlorure d'hafnium, le bromure d'hafnium, le nitrate d'hafnium et les carboxylates d'hafnium comme l'acétate d'hafnium, le propionate d'hafnium, l'adipate d'hafnium, le glutarate d'hafnium, le succinate d'hafnium.

9. Procédé selon l'une des revendications 1 à 8, **caractérisé en ce que** le rapport molaire entre le chrome, le manganèse est compris 0,00001 et 100, de préférence entre 0.001 et 10.

10. Procédé selon l'une des revendications 1 à 9, **caractérisé en ce que** la quantité de catalyseur, exprimée en pourcentage pondéral d'élément manganèse, d'élément chrome et éventuellement d'élément zirconium par rapport au mélange réactionnel, se situe entre 0,0001 et 5 %, de préférence entre 0,001 et 1 %.

11. Procédé selon l'une des revendications 1 à 10, **caractérisé en ce que** le milieu réactionnel liquide contient un solvant choisi parmi les acides carboxyliques aliphatiques ayant de 2 à 9 atomes de carbone, les acides perfluoroalkylcarboxyliques, les alcools, les hydrocarbures halogénés, les cétones, les esters d'alkyle inférieur d'acides carboxyliques, de préférence des acides carboxyliques aliphatiques ayant de 2 à 9 atomes de carbone ou des acides perfluoroalkylcarboxyliques, la tétraméthylène sulfone (ou sulfolane), l'acétonitrile.

12. Procédé selon l'une des revendications 1 à 11, **caractérisé en ce que** le solvant utilisé est l'acide acétique.

13. Procédé selon l'une des revendications 1 à 12, **caractérisé en ce que** le solvant représente de 1 % à 99 % en poids du milieu réactionnel, de préférence de 10 % à 90.

14. Procédé selon l'une des revendications 1 à 13, **caractérisé en ce que** la température à laquelle est réalisée la réaction d'oxydation est comprise entre 50°C et 200°C et de préférence entre 80 °C et 140°C.

15. Procédé selon l'une des revendications 1 à 14, **caractérisé en ce que** la pression à laquelle est réalisée la réaction d'oxydation est comprise entre 0,1 MPa (1 bar) et 20 MPa (200 bar).

## Claims

1. Process for the oxidation of cycloalkanes, cycloalkanols and/or cycloalkanones into carboxylic acid, using oxygen or a gas containing it, in the liquid phase in a solvent chosen from polar protic solvents and polar aprotic solvents and in the presence of a catalyst dissolved in the reaction medium, **characterized in that** the catalyst comprises a soluble manganese compound and a soluble chromium compound.

2. Process according to Claim 1, **characterized in that** the cycloalkane used as starting substrate is chosen from cycloalkanes which have a ring having from 5 to 12 carbon atoms, and is preferably cyclohexane.

3. Process according to Claim 1, **characterized in that** the cycloalkanols and/or the cycloalkanones used as starting substrate are chosen from those which have a ring having from 5 to 12 carbon atoms, and are preferably cyclohexanol and/or cyclohexanone.

4. Process according to one of Claims 1 to 3, **characterized in that** the catalyst comprises at least one manganese compound which is soluble in the reaction medium, chosen from manganese chloride, manganese bromide, manganese nitrate and manganese carboxylates such as manganese acetate tetrahydrate, manganese propionate, manganese adipate, manganese glutarate, manganese succinate.

5. Process according to one of Claims 1 to 4, **characterized in that** the catalyst comprises at least one chromium compound which is soluble in the reaction medium, chosen from chromium chloride, chromium bromide, chromium nitrate and chromium carboxylates such as chromium acetate, chromium propionate, chromium adipate, chromium glutarate and chromium succinate.

6. Process according to one of the preceeding claims, **characterized in that** the catalyst comprises a soluble zirconium or hafnium compound.

7. Process according to Claim 6, **characterized in that** the zirconium compound which is soluble in the reaction medium is chosen from zirconium chloride, zirconium bromide, zirconium nitrate and zirconium carboxylates such as zirconium acetate, zirconium propionate, zirconium adipate, zirconium glutarate and zirconium succinate.

8. Process according to Claim 6, **characterized in that** the hafnium compound which is soluble in the reaction medium is chosen from hafnium chloride, hafnium bromide, hafnium nitrate, hafnium carboxylates such as hafnium acetate, hafnium propionate, hafnium adipate, hafnium glutarate and hafnium succinate.

9. Process according to one of Claims 1 to 8, **characterized in that** the molar ratio between the chromium and the manganese is between 0.00001 and 100 and preferably between 0.001 and 10.

10. Process according to one of Claims 1 to 9, **characterized in that** the amount of catalyst, expressed as a weight percentage of elemental manganese, elemental chromium and possibly of elemental zirconium relative to the reaction mixture, is between 0.0001% and 5% and preferably between 0.001% and 1%.

11. Process according to one of Claims 1 to 10, **characterized in that** the liquid reaction medium contains a solvent chosen from aliphatic carboxylic acids having from 2 to 9 carbon atoms, perfluoroalkylcarboxylic acids, alcohols, halogenated hydrocarbons, ketones, lower alkyl esters of carboxylic acids, preferably aliphatic carboxylic acids having from 2 to 9 carbon atoms or perfluoroalkylcarboxylic acids, tetramethylene sulphone (or sulpholane) and acetonitrile.

12. Process according to one of Claims 1 to 11, **characterized in that** the solvent used is acetic acid.

13. Process according to one of Claims 1 to 12, **characterized in that** the solvent represents from 1% to 99% by weight of the reaction medium, preferably from 10% to 90%.

14. Process according to one of Claims 1 to 13, **characterized in that** the temperature at which the oxidation reaction is carried out is between 50°C and 200°C and preferably between 80°C and 140°C.

15. Process according to one of Claims 1 to 14, **characterized in that** the pressure at which the oxidation reaction is carried out is between 0.1 MPa (1 bar) and 20 MPa (200 bar).

## Patentansprüche

1. Verfahren zur Oxidation von Cycloalkanen, Cycloalkoholen und/oder Cycloketonen zu Carbonsäuren mit Hilfe von Sauerstoff oder eines Sauerstoff enthaltenden Gases in flüssiger Phase in einem unter den polaren protischen und den polaren aprotischen Lösungsmitteln ausgewählten Lösungsmittel und in Gegenwart eines in dem Reaktionsmedium gelösten Katalysators, **dadurch gekennzeichnet, dass** der Katalysator eine lösliche Manganverbindung und eine lösliche Chromverbindung enthält.

2. Verfahren nach Anspruch 1, **dadurch gekennzeichnet, dass** das als Ausgangsverbindung eingesetzte Cycloalkan unter den Cycloalkanen ausgewählt ist, die einen Ring mit 5 bis 12 Kohlenstoffatomen enthalten, und vorzugsweise Cyclohexan ist.

3. Verfahren nach Anspruch 1, **dadurch gekennzeichnet, dass** die als Ausgangsverbindungen eingesetzten Cycloalkohole und Cycloketone unter denjenigen Verbindungen ausgewählt sind, die einen Ring mit 5 bis 12 Kohlenstoffatomen enthalten, und vorzugsweise Cyclohexanol und/oder Cyclohexanon sind.

4. Verfahren nach einem der Ansprüche 1 bis 3, **dadurch gekennzeichnet, dass** der Katalysator mindestens eine in dem Reaktionsmedium lösliche Manganverbindung enthält, die unter Manganchlorid, Manganbromid, Mangannitrat und den Mangancarboxylaten, wie Manganacetat-Tetrahydrat, Manganpropionat, Manganadipat, Manganglutarat und Mangansuccinat, ausgewählt ist.

5. Verfahren nach einem der Ansprüche 1 bis 4, **dadurch gekennzeichnet, dass** der Katalysator mindestens eine in dem Reaktionsmedium lösliche Chromverbindung enthält, die unter Chromchlorid, Chrombromid, Chromnitrat und den Chromcarboxylaten, wie Chromacetat, Chrompropionat, Chromadipat, Chromglutarat und Chromsuccinat, ausgewählt ist.

6. Verfahren nach einem der vorhergenden Anprüche, **dadurch gekennzeichnet, dass** der Katalysator eine lösliche Zirkonium- oder Hafniumverbindung enthält.

7. Verfahren nach Anspruch 6, **dadurch gekennzeichnet, dass** die in dem Reaktionsmedium lösliche Zirkoniumverbindung unter Zirkoniumchlorid, Zirkoniumbromid, Zirkoniumnitrat und den Zirkoniumcarboxylaten, wie Zirkoniumacetat, Zirkoniumpropionat, Zirkoniumadipat, Zirkoniumglutarat und Zirkoniumsuccinat, ausgewählt ist.

8. Verfahren nach Anspruch 6, **dadurch gekennzeichnet, dass** die in dem Reaktionsmedium lösliche Hafniumverbindung unter Hafniumchlorid, Hafniumbromid, Hafniumnitrat und den Hafniumcarboxylaten, wie Hafniumacetat, Hafniumpropionat, Hafniumadipat, Hafniumglutarat und Hafniumsuccinat, ausgewählt ist.

9. Verfahren nach einem der Ansprüche 1 bis 8, **dadurch gekennzeichnet, dass** das Molverhältnis *Chrom*/*Mangan* im Bereich von 0,00001 bis 100 und vorzugsweise von 0,001 bis 10 liegt.

10. Verfahren nach einem der Ansprüche 1 bis 9, **dadurch gekennzeichnet, dass** die Katalysatormenge, ausgedrückt als der auf das Reaktionsgemisch bezogene prozentuale Gesamtgewichtsanteil der Elemente Mangan, Chrom sowie gegebenenfalls Zirkonium im Bereich von 0,0001 bis 5% und vorzugsweise von 0,001 bis 1 % liegt.

11. Verfahren nach einem der Ansprüche 1 bis 10, **dadurch gekennzeichnet, dass** das flüssige Reaktionsmedium ein unter den aliphatischen Carbonsäuren mit 2 bis 9 Kohlenstoffatomen, den Perfluoralkylcarbonsäuren, den Alkoholen, den halogenierten Kohlenwasserstoffen, den Ketonen, den niederen Alkylestern von Carbonsäuren, und zwar vorzugsweise von aliphatischen Carbonsäuren mit 2 bis 9 Kohlenstoffatomen oder von Perfluoralkylcarbonsäuren, Tetramethylensulfon (Sulfolan) und Acetonitril ausgewähltes Lösungsmittel enthält.

12. Verfahren nach einem der Ansprüche 1 bis 11, **dadurch gekennzeichnet, dass** als Lösungsmittel Essigsäure verwendet wird.

13. Verfahren nach einem der Ansprüche 1 bis 12, **dadurch gekennzeichnet, dass** der Gewichtsanteil des Lösungsmittels am Reaktionsmedium 1 bis 99 % und vorzugsweise 10 bis 90 % beträgt.

14. Verfahren nach einem der Ansprüche 1 bis 13, **dadurch gekennzeichnet, dass** die Temperatur, bei der die Oxidationsreaktion durchgeführt wird, im Bereich von 50 bis 200 °C und vorzugsweise von 80 bis 140 °C liegt.

15. Verfahren nach einem der Ansprüche 1 bis 14, **dadurch gekennzeichnet, dass** der Druck, bei dem die Oxidationsreaktion durchgeführt wird, im Bereich von 0,1 MPa (1 bar) bis 20 MPa (200 bar) liegt.
